# EUROPÄISCHE PATENTSCHRIFT

(11) **EP 3 522 993 B1**
(45) Veröffentlichungstag und Bekanntmachung des Hinweises auf die Patenterteilung: **07.04.2021**
(21) Anmeldenummer: 17786842.9
(22) Anmeldetag: 02.10.2017
(51) Int. Cl.: A61Q 19/04, A61K 8/42

(54) **MONOSUBSTITUIERTE HARNSTOFFDERIVATE ALS SELBSTBRÄUNGSSUBSTANZ**
MONOSUBSTITUTED UREA DERIVATIVES AS A SELF-TANNING SUBSTANCE
DÉRIVÉS D'URÉE MONOSUBSTITUÉS UTILISÉS EN TANT QUE SUBSTANCE AUTO-BRONZANTE

(30) Priorität: 07.10.2016 DE 102016011953
(43) Veröffentlichungstag der Anmeldung: 14.08.2019
(73) Patentinhaber: Merck Patent GmbH, 64293 Darmstadt (DE)
(72) Erfinder: OSTHOFF, Julian, 64807 Dieburg (DE); DRILLER, Hansjuergen, 64823 Gross-Umstadt (DE); CAROLA,Christophe, 64625 Bensheim (DE); BACK, Robin, 64625 Bensheim (DE); KROHN, Michael, 64653 Lorsch (DE)
(86) Internationale Anmeldenummer: PCT/EP2017/074963
(87) Internationale Veröffentlichungsnummer: WO 2018/065358

(56) Entgegenhaltungen:
- EP-A1- 1 454 613
- EP-A1- 1 454 615
- WO-A2-2007/013811
- WO-A2-2014/174075
- FR-A1- 3 004 934
- US-A1- 2004 231 069
- US-B1- 6 248 746
- CRITON M ET AL: "Analogues of N-hydroxy-N'-phenylthiourea and N-hydroxy-N'-phenylurea as inhibitors of tyrosinase and melanin formation", BIOORGANIC & MEDICINAL CHEMISTRY LETTERS, PERGAMON, AMSTERDAM, NL, Bd. 18, Nr. 12, 15. Juni 2008 (2008-06-15) , Seiten 3607-3610, XP022707442, ISSN: 0960-894X, DOI: 10.1016/J.BMCL.2008.04.079 [gefunden am 2008-05-04]
- DAVID A BROWN: "Skin pigmentation enhancers", JOURNAL OF PHOTOCHEMISTRY AND PHOTOBIOLOGY B: BIOLOGY, Bd. 63, Nr. 1-3, 1. Oktober 2001 (2001-10-01), Seiten 148-161, XP055085922, ISSN: 1011-1344, DOI: 10.1016/S1011-1344(01)00212-3

## Beschreibung

Die vorliegende Erfindung betrifft die Verwendung von monosubstituierten Harnstoffderivaten der Formel I als Selbstbräunungssubstanz, zur Steigerung der Melaninsynthese, zur Verbesserung des Melanintransports und/oder Verbesserung der Verteilung von Melanin in suprabasalen Schichten, sowie Zubereitungen enthaltend diese Harnstoffderivate.

Der Trend weg von der vornehmen Blässe hin zur "gesunden, sportlich braunen Haut" ist seit Jahren ungebrochen. Um einen gebräunten Teint zu erzielen, setzen die Menschen ihre Haut der Sonnenstrahlung aus, da diese eine Pigmentierung durch eine Melaninbildung hervorruft. Die UV-Strahlung des Sonnenlichtes hat jedoch auch eine schädigende Wirkung auf die Haut. Neben der akuten Schädigung (Sonnenbrand) treten Langzeitschäden bei übermäßiger Bestrahlung mit Licht aus dem UVB-Bereich (Wellenlänge 280-320 nm) auf, wie beispielsweise ein erhöhtes Risiko, an Hautkrebs zu erkranken. Die übermäßige Einwirkung der UVB- und UVA-Strahlung (Wellenlänge: 320-400 nm) erzeugt hochreaktive Radikalspezies, die sich auch nach Beendigung der Bestrahlung weiter vermehren und als deren Folge es zu Faltenbildung und Hautalterung kommt.

Den natürlichen Schutz vor den negativen Folgen der Sonnenstrahlung bietet die Bräunung (Pigmentierung) der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantinocyten (Hornzellen) transportiert und dort als braune Hautfarbe sichtbar wird. Melanin schützt die Zellkerne vor weiterer Bestrahlung und den davon verursachten negativen Auswirkungen auf die Zell-DNA.

Je nach chemischer Zusammensetzung der biochemisch gebildeten Pigmente wird zwischen dem bräunlich-schwarzen Eumelanin und dem rötlich-gelben Pheomelanin unterschieden. Der beobachtete Hautfarbton wird vom Verhältnis dieser beiden Melaninarten bestimmt.
Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen. Bei der "Direkt-Pigmentierung", die mit der Sonnenbestrahlung einsetzt, werden vorwiegend farblose Melanin-Vorstufen durch UVA-Strahlung zu dunkel gefärbtem Melanin oxidiert. Da diese Oxidierung reversibel ist, führt sie zu einer nur kurz anhaltenden Hautbräunung.

Eine künstliche Bräunung der Haut lässt sich äußerlich mit Hilfe von Schminke und oral durch Einnahme von Carotinoiden erzeugen.

Weitaus beliebter jedoch ist die künstliche Bräunung der Haut, welche sich durch Auftragen von sogenannten Selbstbräunern erzielen lässt.
Diese Verbindungen weisen als chemisches Strukturmerkmal Keto- bzw. Aldehydgruppen in Nachbarschaft zu Alkoholfunktionen auf und gehören überwiegend zur Substanzklasse der Zucker. Besonders häufig eingesetzte Selbstbräunungssubstanzen sind 1,3-Dihydroxyaceton (DHA), das in einer Menge von 700t/a verwendet wird, und Erythrulose.

Selbstbräuner können mit den Proteinen und Aminosäuren der Hornschicht der Haut im Sinne einer Maillard-Reaktion oder über eine Michael Addition umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen. Die so erzielte Bräune ist nicht abwaschbar und wird erst mit der normalen Hautabschuppung entfernt.

Diese Farbprodukte besitzen allerdings selbst keine UV-absorbierenden Eigenschaften, sodass bei Sonnenexposition ein zusätzlicher Sonnenschutz (Bekleidung, Hut, Auftragen von UV-Filtern) erforderlich wird.
Im Gegensatz zu "sonnengebräunter" Haut ist die so gebräunte Haut nicht gegen Sonnenbrand geschützt.

Es besteht daher weiterhin ein Bedarf nach dermatologisch verträglichen hautfärbenden Substanzen, die sich zum Einsatz in kosmetischen und/oder dermatologischen Zubereitungen oder Medizinprodukten eignen und die die natürliche Bräunung der Haut durch Steigerung der Melaninsynthese verstärken und gleichzeitig einen besseren Hauteigenschutz bzw. Sonnenschutz, insbesondere gegen UVB-Strahlung, ermöglichen.

Die der vorliegenden Erfindung zugrunde liegende Aufgabe bestand daher in der Bereitstellung von neuen Selbstbräunungssubstanzen mit verbesserten Eigenschaften.

Überraschenderweise wurde nun festgestellt, dass sich bestimmte monosubstituierte Harnstoffderivate als Selbstbräunungssubstanzen eignen, insbesondere als Selbstbräunungssubstanzen, die die natürliche Bräunung der Haut durch Einwirkung auf die Melaninsynthese und/oder Melaninverteilung ermöglichen.

Im Sinne der Erfindung wird der Begriff Selbstbräunungswirkstoff synonym zu Selbstbräunungssubstanz oder Selbstbräunersubstanz verwendet.

Aus Marc Criton et al, Bioorganic & Medicinal Chemistry Letters 2008, 18, 3607-3610 sind strukturell verwandte Verbindungen des N-Hydroxy-N'-Phenylthioharnstoffs und des N-Hydroxy-N'-Phenylharnstoffs bekannt, die auch als Hautaufheller, insbesondere als Tyrosinaseinhibitoren, beschrieben sind.

Ein erster Gegenstand der vorliegenden Erfindung ist daher die Verwendung von Verbindungen der Formel I, 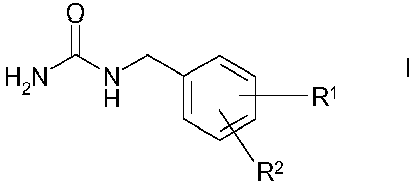 wobei
R¹ und R² unabhängig voneinander für
   -H,
   -OH,
   -geradkettiges oder verzweigtes O-(C₁- bis C₆-Alkyl) stehen,
wobei R¹ und R² auch zusammen einen unsubstituierten oder substituierten fünfgliedrigen Ring bilden können, wobei ein oder zwei nicht benachbarte CH₂-Gruppen durch O ersetzt sein können und der durch mindestens eine geradkettige oder verzweigte Alkylgruppe mit 1 bis 6 C-Atomen substituiert sein kann,
und/oder deren Salze, Tautomere, Konformere und/oder Solvate, einschließlich deren Mischungen in allen Verhältnissen, als Selbstbräunungssubstanz.

Ein weiterer Gegenstand der Erfindung sind Verbindungen der Formel I, 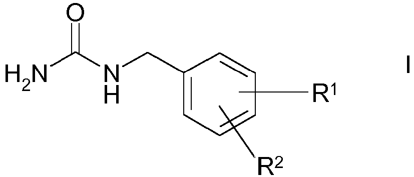 wobei
R¹ und R² unabhängig voneinander für
   -H,
   -OH,
   -geradkettiges oder verzweigtes O-(C₁- bis C₆-Alkyl) stehen,
wobei R¹ und R² auch zusammen einen unsubstituierten oder substituierten fünfgliedrigen Ring bilden können, wobei ein oder zwei nicht benachbarte CH₂-Gruppen durch O ersetzt sein können und der durch mindestens eine geradkettige oder verzweigte Alkylgruppe mit 1 bis 6 C-Atomen substituiert sein kann,
und/oder deren Salze, Tautomere, Konformere und/oder Solvate, einschließlich deren Mischungen in allen Verhältnissen, zur Verwendung zur Steigerung der Melaninsynthese, zur Verbesserung des Melanintransports und/oder zur Verbesserung der Verteilung von Melanin in suprabasalen Schichten.

Im Rahmen der Erfindung sind die Verbindungen der Formel I, so definiert, dass man darunter auch pharmazeutisch oder kosmetisch verwendbare Salze, Hydrate, Solvate, Tautomere und Konformere versteht. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Zu den bevorzugten Salzen gehören dabei insbesondere Alkali- und Erdalkalimetallsalze, Zink- sowie Ammonium-Salze, insbesondere jedoch Natrium- und Kalium-Salze.

Als Tautomere bezeichnet man Moleküle mit gleicher Summenformel, deren Atome unterschiedlich verknüpft sind, die durch die Wanderung einzelner Atome oder Atomgruppen schnell ineinander übergehen, d. h. die beiden Isomere in einem schnellen chemischen Gleichgewicht miteinander stehen.

Die Konformation eines organischen Moleküls beschreibt die räumliche Anordnung dessen drehbarer Bindungen an den Kohlenstoffatomen. Durch sie sind die dreidimensionalen Raumkoordinaten aller Atome des Moleküls vollständig beschrieben. Moleküle mit gleicher Anordnung von Atomen, die sich jedoch in der spezifischen Anordnung der Atome unterscheiden und in einem Energieminimum liegen, bezeichnet man als Konformere. Synonym dazu ist auch die Bezeichnung Rotamer gebräuchlich.

Eine geradkettige oder verzweigte Alkylgruppe mit 1 bis 6 C-Atomen ist beispielsweise Methyl, Ethyl, iso-Propyl, n-Propyl, n-Butyl, sec-Butyl oder *tert*-Butyl, Pentyl, 1-, 2- or 3-Methylbutyl, 1,1-, 1,2- or 2,2-Dimethylpropyl, 1-Ethylpropyl oder n-Hexyl.

Der vorliegend verwendete Terminus "C₁- bis C₆-Alkyl" bedeutet eine geradkettige oder verzweigte Alkylgruppe mit 1 bis 6 C-Atomen, wie zuvor beschrieben.

Der vorliegend verwendete Terminus "O-(C₁- bis C₆-Alkyl)" bedeutet eine geradkettige oder verzweigte Alkoxygruppe mit 1 bis 6 C-Atomen, wobei die zuvor beschriebene Alkylgruppe entsprechend an ein O-Atom gebunden vorliegt.

Verbindungen der Formel I werden bevorzugt verwendet, wenn der Substituent R¹ in Formel I bevorzugt für H, OH oder eine geradkettige oder verzweigte Alkoxygruppe mit 1 bis 4 C-Atomen steht. Der Substituent R¹ in Verbindungen der Formel I steht besonders bevorzugt für H, OH oder OCH₃.

Ein weiterer Gegenstand der Erfindung ist daher die Verwendung von Verbindungen der Formel I, wie zuvor beschrieben, wobei der Substituent R¹ für H, OH oder eine geradkettige oder verzweigte Alkoxygrupe mit 1 bis 4 C-Atomen steht, oder bevorzugt für H, OH oder OCH₃ steht.

Verbindungen der Formel I werden bevorzugt verwendet, wenn der Substituent R² in Formel I für H, OH oder eine geradkettige oder verzweigte Alkoxygruppe mit 1 is 4 C-Atomen steht. Der Substituent R² in Verbindungen der Formel I steht besonders bevorzugt für H, OH oder OCH₃.

Ein weiterer Gegenstand der Erfindung ist daher die Verwendung von Verbindungen der Formel I, wie zuvor beschrieben, wobei der Substituent R² für H, OH oder eine geradkettige oder verzweigte Alkoxygrupe mit 1 bis 4 C-Atomen steht, oder bevorzugt für H, OH oder OCH₃ steht.

Wie zuvor beschrieben, können die Substituenten R¹ und R² in Verbindungen der Formel I zusammen einen fünfgliedrigen Ring bilden wobei ein oder zwei nicht benachbarte CH₂-Gruppen durch O ersetzt sein können und der durch mindestens eine geradkettige oder verzweigte Alkylgruppe mit 1 bis 6 C-Atomen substituiert sein kann.

In einer besonders bevorzugten Ausführungsform der vorliegenden Erfindung werden Verbindungen der Formel I verwendet, in denen die Substituenten R¹ und R² in Formel I zusammen bevorzugt einen fünfgliedrigen Ring enthaltend zwei O-Atome bilden, der durch eine oder zwei geradkettige oder verzweigte Alkylgruppe(n) mit 1 bis 6 C-Atomen substituiert sein kann. Bevorzugt ist ein derartig gebildeter fünfgliedriger Ring mit einer oder zwei Methylgruppe(n) substituiert oder unsubstituiert.

Ein weiterer Gegenstand der Erfindung ist daher die Verwendung von Verbindungen der Formel I, wie zuvor beschrieben, wobei die Substituenten R¹ und R² zusammen einen fünfgliedrigen Ring enthaltend zwei O-Atome bilden, der durch eine oder zwei geradkettige oder verzweigte Alkylgruppe(n) mit 1 bis 6 C-Atomen substituiert sein kann.

Der Substituent R¹ steht bevorzugt an Position 3 des Benzylrings; der Substituent R² steht bevorzugt an Position 4 des Benzylrings, visualisiert durch die Formel I-1: 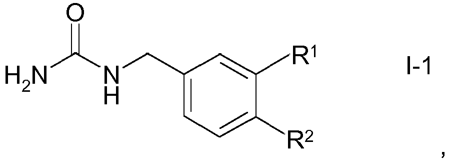 wobei R¹ und R² eine jeweils zuvor genannte Bedeutung oder bevorzugt genannte Bedeutung haben.

Beispiele für Verbindungen der Formel I bzw. der Formel I-1 sind die Verbindungen Ia bis Ik:

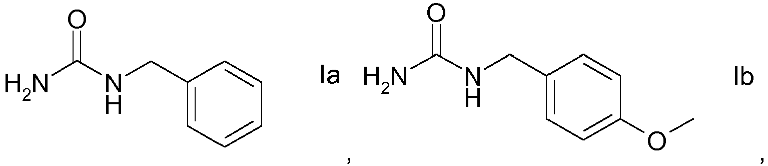

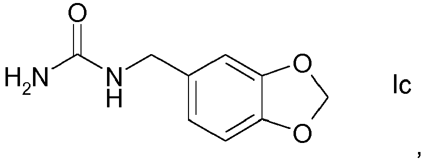

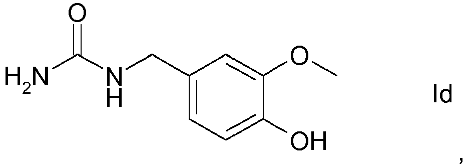

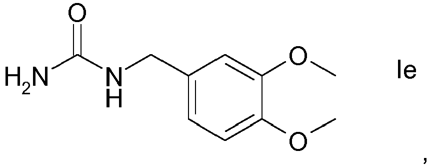

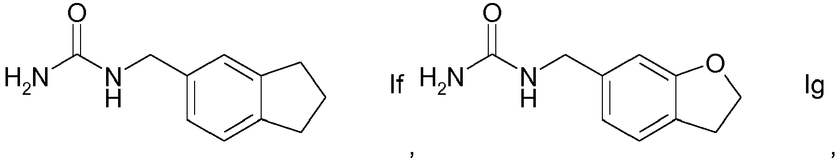

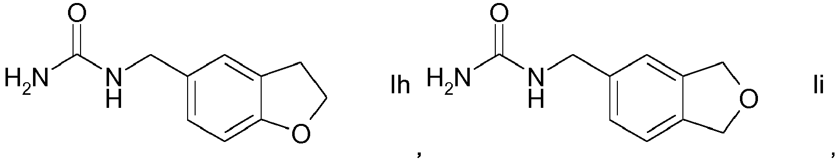

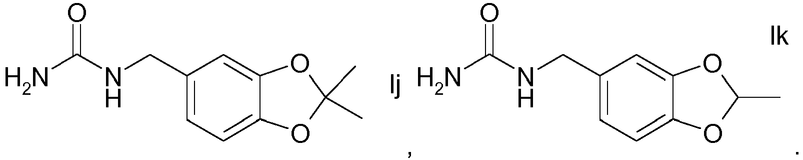

Besonders bevorzugt wird zur erfindungsgemäßen Verwendung, wie zuvor beschrieben, die Verbindung der Formel I aus den Verbindungen der Formel Ia, Ib, Ic, Id und le ausgewählt, 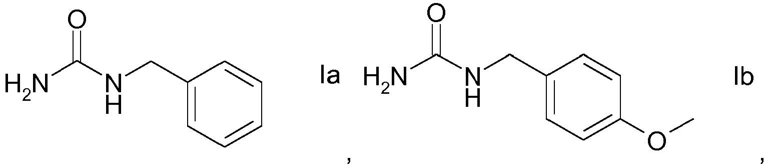 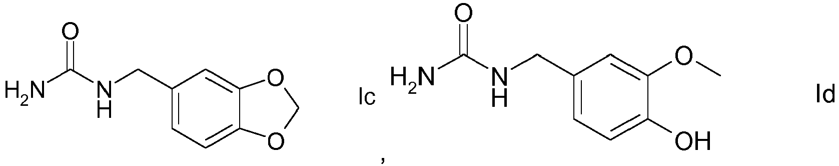 und

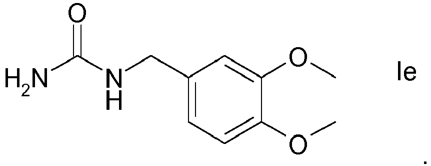

Ganz besonders bevorzugt wird zur erfindungsgemäßen Verwendung, wie zuvor beschrieben, mindestens eine Verbindung der Formel Ia, Ib und/oder Ic ausgewählt. Aus der Gruppe der Verbindungen der Formel Ia, Ib und/oder Ic ist die Verbindung der Formel Ic bevorzugt.

Die Verbindungen der Formel I sind kommerziell erhältlich oder können nach Synthesen hergestellt werden, die dem Fachmann bekannt sind. Sie können beispielsweise durch Reaktion eines entsprechenden Amins der Formel II 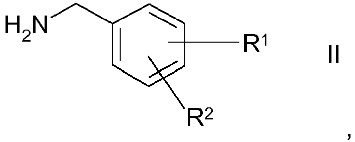 wobei die Substituenten R¹ und R² eine der zuvor angegebenen oder bevorzugt angegebnen Bedeutungen haben, mit Harnstoff unter Säurekatalyse hergestellt werden.
Die dazu notwendige Reaktionsbedingung findet ein Fachmann auf dem Gebiet der organischen Synthese problemlos in der allgemein zugänglichen Literatur zu organischen Reaktionen. Beispiele für Reaktionsbedingungen sind im Ausführungsteil beschrieben.

Bevorzugt findet die Reaktion in Wasser statt. Wahlweise kann in organischen Lösemitteln oder in Mischungen von organischen Lösemitteln mit Wasser umgesetzt werden.

Die Reaktion erfolgt vorzugsweise unter Säurekatalyse mit konzentrierter Salzsäure. Die Reaktionstemperatur liegt zwischen 60°C und 130°C. Besonders bevorzugt wird bei der Siedetemperatur des Lösemittel oder Lösemittelgemisches gearbeitet. Oft schließt sich der Umsetzung eine geeignete Aufreinigung an.

Geeignete Aufreinigungsschritte umfassen das Abtrennen von leicht flüchtigen Komponenten durch Destillation oder Kondensation, eine Extraktion mit einem organischen Lösungsmittel, eine Fällung durch Zugabe eines organischen Lösemittels, einer Umsalzung oder eine Kombination dieser Methoden. Jede bekannte Trennmethode kann hierfür verwendet oder kombiniert werden. In der Regel fällt das gewünschte Reaktionsprodukt aus der Reaktionsmischung aus und wird entsprechend abgetrennt und aufgereinigt.
Weitere Details sind in den Beispielen aufgeführt, die entsprechend auch für die allgemeine Synthesebeschreibung gelten.

Verbindungen der Formel I steigern überraschenderweise die Melaninsynthese und/oder verbessern den Melanintransport von den Melanocyten zu den Keratinocyten und/oder verteilen Melanin besser in suprabasalen Schichten. Eine Verbindung der Formel I ist daher auch als Melanogenese-Promotor oder Propigmentierungswirkstoff zu bezeichnen. Die Verbindungen der Formel I steigern bevorzugt die Melaninsynthese. Dies wirkt sich auf die Farbe der Haut aus und bewirkt einen Bräunungseffekt. Diese Eigenschaft ist insofern überraschend, da für ähnliche Verbindungen der entgegengesetzte Effekt, nämlich eine Inhibierung der Melaninsynthese beschrieben wird.

Die Verbindungen der Formel I haben neben der Bräunungswirkung auch eine gute Hautverträglichkeit. Zusätzlich sind bevorzugte der hier beschriebenen Verbindungen farblos oder nur schwach gefärbt und führen so nicht oder nur in geringer Weise zu Verfärbungen der Zubereitungen. Die bevorzugten Verbindungen weisen außerdem eine verbesserte Löslichkeit in kosmetischen Ölen auf.

Damit die Verbindungen der Formel I ihre positive Wirkung auf die Haut besonders gut entwickeln können, kann es bevorzugt sein die Verbindungen der Formel I, wie zuvor beschrieben, in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der topischen Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar.

Die erfindungsgemäßen Verwendungen erfolgen bevorzugt nichttherapeutisch.

Ein weiterer Gegenstand der vorliegenden Erfindung ist eine Zubereitung enthaltend einen für topische Anwendungen geeigneten Träger, mindestens eine Verbindung der Formel I in einer Menge von 0,01 bis 10 Gew.-%, 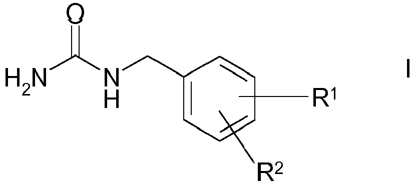 wobei
R¹ und R² unabhängig voneinander für
   -H,
   -OH,
   -geradkettiges oder verzweigtes O-(C₁- bis C₆-Alkyl) stehen,
wobei R¹ und R² auch zusammen einen unsubstituierten oder substituierten fünfgliedrigen Ring bilden können, wobei ein oder zwei nicht benachbarte CH₂-Gruppen durch O ersetzt sein können und der durch mindestens eine geradkettige oder verzweigte Alkylgruppe mit 1 bis 6 C-Atomen substituiert sein kann,
und/oder deren Salze, Tautomere, Konformere und/oder Solvate, einschließlich deren Mischungen in allen Verhältnissen, oder eine bevorzugte Verbindung der Formel I, wie zuvor beschrieben und mindestens eine weitere Selbstbräunungssubstanz.

Bei der topisch anwendbaren Zubereitung handelt es sich dabei üblicherweise um kosmetische oder dermatologische Formulierungen oder Medizinprodukte. Die Zubereitungen enthalten in diesem Fall einen kosmetisch oder dermatologisch geeigneten Träger oder einen für ein Medizinprodukt geeigneten Träger und je nach gewünschtem Eigenschaftsprofil optional weitere geeignete Inhaltsstoffe.

Im Sinne der vorliegenden Erfindung wird neben dem Begriff Zubereitung gleichbedeutend auch der Begriff Mittel oder Formulierung verwendet.

Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können.

Die Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Bevorzugt handelt es sich um eine kosmetische oder dermatologische Zubereitung; besonders bevorzugt handelt es sich um eine kosmetische Zubereitung.

Die mindestens eine Verbindung der Formel I, wie zuvor beschrieben oder als bevorzugt beschrieben, wird in den erfindungsgemäßen Zubereitungen typischerweise in Mengen von 0,01 bis 10 Gew.-%, bevorzugt in Mengen von 0,05 bis 10 Gew.-%, besonders bevorzugt in Mengen von 0,1 Gew.-% bis 5 Gew.-% und ganz besonders bevorzugt in Mengen von 0,5 bis 2 Gew.-%, bezogen auf die Gesamtmenge der Zubereitung, eingesetzt. Dabei bereitet es dem Fachmann keinerlei Schwierigkeiten die Mengen abhängig von der beabsichtigten Wirkung der Zubereitung entsprechend auszuwählen.

Ferner enthalten die erfindungsgemäßen Zubereitungen mindestens eine weitere Selbstbräunungssubstanz als weiteren Inhaltsstoff. Dabei kann es sich sowohl um einen Selbstbräuner handeln, der mit den Aminosäuren der Haut im Sinne einer Maillard-Reaktion oder über eine Michael-Addition reagiert, als auch um einen sogenannten Melanogenese-Promotor oder Propigmentierungswirkstoff, der die natürliche Bräunung der Haut fördert.

Als vorteilhafte Selbstbräunungssubstanzen können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist 1,3-Dihydroxyaceton, Erythrulose oder deren Kombination. Propigmentierungsstoffe können prinzipiell alle dem Fachmann bekannten Wirkstoffe sein. Beispiele hierfür sind Glycyrrhetinsäure, Melanocytenstimulierendes Hormon (alpha-MSH), Peptidanaloga, Thymidin-Dinucleotide, L-Tyrosin und dessen Ester oder bicyclische Monoterpendiole (beschrieben in Brown et al., Photochemistry and Photobiology B: Biology 63 (2001) 148-161) oder Hexadecansäure 5-hydroxy-2-methyl-4-oxo-4H-chromen-7-yl ester, der von der Firma Merck unter dem Handelsnamen RonaCare® Bronzyl™ vermarktet wird. Besonders geeignete Wirkstoffe zur Kombinaton mit mindestens einer Verbindung der Formel I, wie zuvor beschrieben, sind 1,3-Dihydroxyaceton, Erythrulose und/oder Hexadecansäure 5-hydroxy-2-methyl-4-oxo-4H-chromen-7-yl ester. Bevorzugt ist die mindestens eine weitere Selbstbräunungssubstanz in der Zubereitung in einer Menge von 0,01 bis 20 Gew.-%, besonders bevorzugt in einer Menge von 0,1 bis 15 Gew.-% und ganz besonders bevorzugt in einer Menge von 0,2 bis 8 Gew.-%, bezogen auf die Gesamtmenge der Zubereitung, enthalten.

Zubereitungen mit Selbstbräunereigenschaften, insbesondere solche, die Dihydroxyaceton enthalten, neigen bei der Anwendung auf der menschlichen Haut zu Fehlgerüchen, die vermutlich durch Abbauprodukte des Dihydroxyacetons selbst oder durch Produkte von Nebenreaktionen verursacht werden und die von den Anwendern teilweise als unangenehm empfunden werden. Es hat sich gezeigt, dass diese Fehlgerüche bei Verwendung von Formaldehydfängern und/oder Flavonoiden vermieden werden. Daher kann die erfindungsgemäße Zubereitung auch Formaldehydfänger sowie gegebenenfalls Flavonoide zur Verbesserung des Geruches enthalten.

Vorzugsweise wird der Formaldehydfänger ausgewählt aus der Gruppe Alkalimetall-, Erdalkalimetall- oder Ammoniumdisulfit. Besonders bevorzugt ist eine Zubereitung, die in Kombination DHA Plus, eine Mischung aus DHA, Natriumdisulfit und Magnesiumstearat, enthält.

DHA Plus ist eine Produktmischung, welche zur Maskierung, Eliminierung oder Neutralisierung des Formaldehyds Natriummetabisulfit, gleichbedeutend mit Na₂S₂O₅ oder INCI: sodium disulfite, enthält. Der Zusatz von Natriummetabisulfit in fertigen Formulierungen führt zur signifikanten Reduktion oder Unterbindung des unangenehmen Geruchs. DHA Plus wird von der Firma Merck, Darmstadt vertrieben.

Die erfindungsgemäße Zubereitung enthaltend mindestens eine Verbindung der Formel I, wie zuvor mit den angegebenen und auch bevorzugt genannten Substituenten beschrieben, sowie Dihydroxyaceton als Selbstbräuner, kann besonders bevorzugt Flavonoide zur Verbesserung des Geruches und gegebenenfalls zur Bräunungsbeschleunigung enthalten.

Das Flavonoid wirkt dabei zusätzlich als Stabilisator für den Selbstbräuner bzw. die Selbstsbräunungssubstanzen und/oder reduziert oder vermeidet oder verbessert lagerabhängige Fehlgerüche, die auch durch enthaltene Zusatz- oder Hilfsstoffe entstehen können.

Vorzugsweise handelt es sich um ein Flavonoid, bei dem eine oder mehrere phenolische Hydroxygruppen durch Veretherung oder Veresterung blockiert sind. Beispielsweise haben sich Hydroxyethyl-substituierte Flavonoide, wie vorzugsweise Troxerutin, Troxequercetin, Troxeisoquercetin oder Troxeluteolin, und Flavonoid-sulfate oder Flavonoid-phosphate, wie vorzugsweise Rutinsulfate, dabei als besonderes gut geeignete Flavonoide erwiesen. Besonders bevorzugt im Sinne dieser Verwendung sind Rutinsulfat und Troxerutin. Ganz besonders bevorzugt ist die Verwendung von Troxerutin.

Die bevorzugten Flavonoide verfügen über einen nicht positiv geladenen Flavangrundkörper. Es wird vermutet, dass durch diese Flavonoide Metallionen wie z.B. Fe²⁺/Cu²⁺ komplexiert werden und so Autooxidationsvorgänge bei Duftstoffen oder Verbindungen, deren Abbau zu Fehlgerüchen führt, verhindert bzw. vermindert werden.

Besonders bevorzugt ist eine Zubereitung, die neben mindestens einer Verbindung der Formel I, wie zuvor beschrieben oder bevorzugt beschrieben, DHA Rapid enthält. DHA Rapid ist eine Produktmischung enthaltend Dihydroxyaceton und Troxerutin, der Firma Merck, Darmstadt. Diese besonders bevorzugte Zubereitung kann gegebenenfalls auch noch einen Formaldehydfänger, beispielsweise Natriumdisulfit enthalten.

Entsprechende Vormischungen und Zubereitungen, die Formaldehydfänger sowie gegebenenfalls Flavonoide zur Verbesserung des Geruches auf der Haut enthalten, sind in der Deutschen Patentanmeldung DE 10 2007 013 368 A1 beschrieben.

Die erfindungsgemäßen Zubereitungen können auch neben den Verbindungen der Formel I, wie zuvor beschrieben oder als bevorzugt beschrieben, zusätzlich mindestens einen UV-Filter enthalten.

Organische UV-Filter, sogenannte hydrophile oder lipophile Sonnenschutzfilter, sind im UVA-Bereich und/oder UVB-Bereich und/oder IR und/oder VIS-Bereich (Absorber) wirksam. Diese Substanzen können insbesondere unter Dibenzoylmethanderivaten, Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind:
Dibenzoylmethanederivative, beispielsweise 4-Isopropyldibenzoylmethane und 4,4'-Methoxy-tert-butyldibenzoylmethane, welche in den Offenlegungsschriften FR-A-2 326 405, FR-A-2 440 933 und
EP-A-0 114 607 beschrieben sind. 4,4'-Methoxy-tert-butyldibenzoylmethane wird beispielsweise von der Firma Merk unter dem Namen Eusolex 9020 vertrieben.

Para-Aminobenzoesäure und deren Derivate: PABA, Ethyl PABA, Ethyl dihydroxypropyl PABA, Ethylhexyl dimethyl PABA, z. B. vertrieben unter dem Namen "Escalol 507" von der Fa. ISP, Glyceryl PABA, PEG-25 PABA, z. B. vertrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vertrieben unter dem Namen "Neo Heliopan OS" von der Fa. Symrise, Dipropylene glycol salicylate, z. B. vertrieben unter dem Namen "Dipsal" von der Fa. Scher, TEA salicylate, z. B. vertrieben unter dem Namen "Neo Heliopan TS" von der Fa. Symrise.

β,β-Diphenylacrylate Derivate: Octocrylene, z. B. vertrieben unter dem Namen "Eusolex OCR" von der Firma Merck", "Uvinul N539" von der Fa. BASF, Etocrylene, z. B. vertrieben unter dem Namen "Uvinul N35" von der BASF.

Benzophenone Derivate: Benzophenone-1, z. B. vertrieben unter dem Namen "Uvinul 400"; Benzophenone-2, z. B. vertrieben unter dem Namen "Uvinul D50" ; Benzophenone-3 oder Oxybenzone, z. B. vertrieben unter dem Namen "Uvinul M40";Benzophenone-4, z. B. vertrieben unter dem Namen "Uvinul MS40" ; Benzophenone-9, z. B. vertrieben unter dem Namen "Uvinul DS-49" von der Fa. BASF, Benzophenone-5, Benzophenone-6, z. B. vertrieben unter dem Namen "Helisorb 11" von der Fa. Norquay, Benzophenone-8, z. B. vertrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vertrieben unter dem Namen "Mexoryl SD" von der Fa. Chimex, 4-Methylbenzylidenecamphor, z. B. vertrieben unter dem Namen "Eusolex 6300" von der Fa. Merck, Benzylidenecamphorsulfonsäure, z. B. vertrieben unter dem Namen "Mexoryl SL" von der Fa. Chimex, Camphor benzalkonium methosulfate, z. B. vertrieben unter dem Namen "Mexoryl SO" von der Fa. Chimex, Terephthalylidenedicamphorsulfonsäure, z. B. vertrieben unter dem Namen "Mexoryl SX" von der Fa Chimex, Polyacrylamidomethylbenzylidenecamphor vertrieben unter dem Namen "Mexoryl SW" von der Fa. Chimex.

Phenylbenzimidazol Derivate: Phenylbenzimidazolesulfonsäure, z. B. vertrieben unter dem Namen "Eusolex 232" von der Fa. Merck, Dinatrium phenyl dibenzimidazole tetrasulfonat, z. B. vertrieben unter dem Namen "Neo Heliopan AP" von der Fa. Symrise.

Phenylbenzotriazol Derivate: Drometrizole trisiloxane, z. B. vertrieben unter dem Namen "Silatrizole" von der Fa. Rhodia Chimie, Methylenebis(benzotriazolyl)tetramethylbutylphenol in fester Form, z. B. vertrieben unter dem Namen "MIXXIM BB/100" von der Fa. Fairmount Chemical, oder in mikronisierter Form als wässrige Dispersion, z. B. vertrieben unter dem Namen "Tinosorb M" von der Fa. BASF.

Triazin Derivate: Ethylhexyltriazone, z. B. vertrieben unter dem Namen "Uvinul T150" von der Fa. BASF, Diethylhexylbutamidotriazone, z. B. vertrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine vertrieben als Tinosorb A2B von BASF, 2,2'-[6-(4-methoxyphenyl)-1,3,5-triazine-2,4-diyl]bis[5-(2-ethylhexyl)oxy]-phenol, vertrieben als Tinosorb S von BASF, N2,N4-bis[4-[5-(1,1-dimethylpropyl)-2-benzoxazolyl]phenyl]-N6-(2-ethylhexyl)-1,3,5-triazin-2,4,6-triamin vertrieben als Uvasorb K 2A von der Firma Sigma 3V.

Anthranilin Derivate: Menthyl anthranilate, z. B. vertrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vertrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vertrieben unter dem Namen Uvasorb K2A von der Fa. Sigma 3V und Mischungen dieses enthaltend.

Piperazinderivate wie beispielsweise die Verbindung 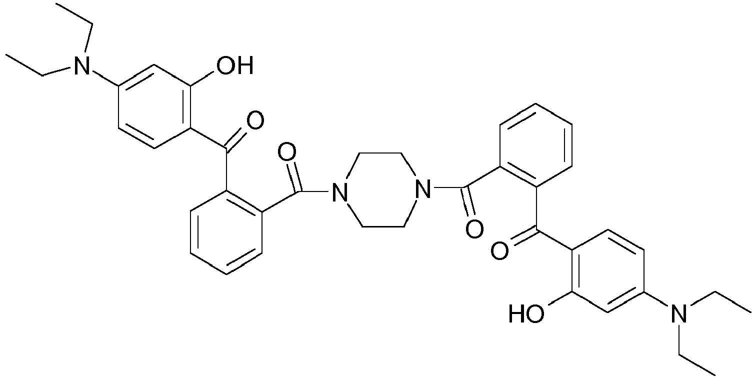 oder die UV-Filter der folgenden Strukturen 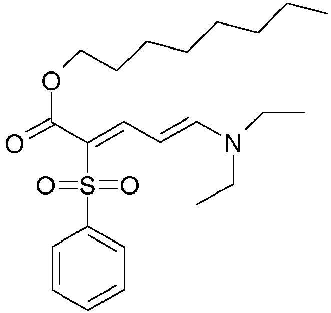 oder

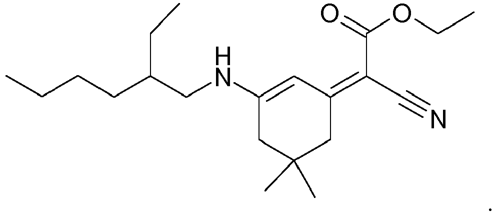

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

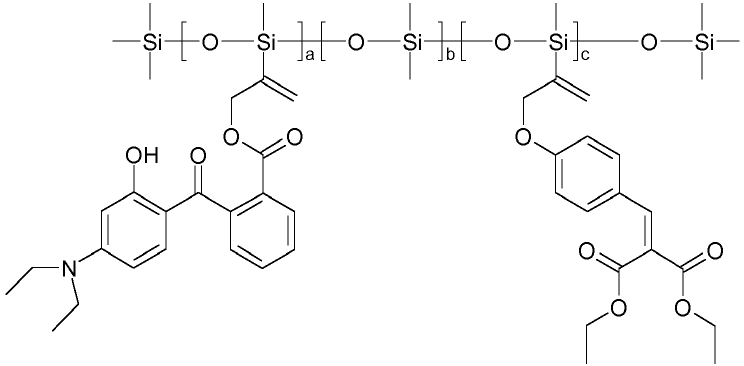

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Butyl Methoxydibenzoylmethane, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Die Zubereitungen können neben den Verbindungen der Formel I sowie den gegebenenfalls organischen UV-Filtern, wie zuvor beschrieben, weitere anorganische UV-Filter, sogenannte partikuläre UV-Filter, enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex® T-2000, Eusolex® T-AQUA, Eusolex®T-AVO, Eusolex®T-PRO, Eusolex® T-EASY), Zinkoxide (z.B. Sachtotec®), Eisenoxide oder auch Ceroxide und/oder Zirkonoxide bevorzugt.

Ferner sind auch Kombinationen mit pigmentärem Titandioxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53 64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

In den beschriebenen Zubereitungen, die erfindungsgemäß mindestens eine Verbindung der Formel I enthalten, können weiterhin auch Farbpigmente enthalten sein, wobei der Schichtaufbau der Pigmente nicht limitiert ist.

Vorzugsweise sollte das Farbpigment bei Einsatz von 0,5 bis 5 Gew.-% hautfarben oder bräunlich sein. Die Auswahl eines entsprechenden Pigments ist für den Fachmann geläufig.

Bevorzugte Zubereitungen können ebenfalls mindestens einen weiteren kosmetischen Wirkstoff enthalten, beispielsweise ausgewählt aus Antioxidantien, Anti-Ageing-, Anti-Falten-, Anti-Schuppen-, Anti-Akne-, Anti-Cellulite-Wirkstoffen, Deodorants oder Vitaminen.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA, Pentasodium ethylenediamin tetramethylen phosphonat und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selen¬methionin), Stilbene und deren Derivate (z.B. Stilbenoxid, trans-Stilbenoxid).

Geeignete Antioxidantien sind auch Verbindungen der Formeln A oder B 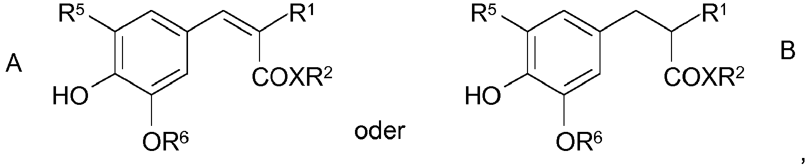 worin
R¹ aus der Gruppe -C(O)CH₃, -CO₂R³, -C(O)NH₂ und -C(O)N(R⁴)₂ ausgewählt werden kann,
X O oder NH,
R² lineares oder verzweigtes Alkyl mit 1 bis 30 C-Atomen,
R³ lineares oder verzweigtes Alkyl mit 1 bis 20 C-Atomen,
R⁴ jeweils unabhängig voneinander H oder lineares oder verzweigtes Alkyl mit 1 bis 8 C-Atomen,
R⁵ H, lineares oder verzweigtes Alkyl mit 1 bis 8 C-Atomen oder lineares oder verzweigtes Alkoxy mit 1 bis 8 C-Atomen und
R⁶ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzyl)-malonsäure-bis-(2-ethylhexyl)ester (z.B. RonaCare® AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L (+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Geeignete Anti-ageing Wirkstoffe, insbesondere für hautpflegende Zubereitungen, sind vorzugsweise sogenannte kompatible Solute. Vorzugsweise werden als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1- Diglycerin-Phosphat (DGP), β-Mannosylglycerat (Firoin), ß- Mannosylglyceramid (Firoin-A), Dimannosyl-di-inositolphosphat (DMIP) oder ein optisches Isomer oder Derivat, z.B. eine Säure, ein Salz oder Ester dieser Verbindungen, und Kombinationen davon.

Additional können als Anti-Ageing Wirkstoffe Produkte der Firma Merck wie z.B. 5,7-Dihydroxy-2-methyl-chromon, vermarktet unter dem Handelsnamen RonaCare®Luremin®, oder die Handelsprodukte RonaCare®ASCIII®, RonaCare®RenouMer, RonaCare®Nicotinamide, RonaCare®VTA, RonaCare®Poppy SE, RonaCare®Isoquercetin oder RonaCare®Cyclopeptide 5 verwendet werden.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-Cellulite-Wirkstoffe. Ein ebenfalls bekannter Anti-Cellulite-Wirkstoff ist Koffein.

Die Zubereitungen können in bevorzugter Weise Hilfsstoffe enthalten, wie beispielsweise kosmetische Öle (z.B. Caprylic/Capric Triglycerides, C12-15 Alkyl Benzoate, Isopropylmyristat, Arylalkyl Benzoate wie z.B. Phenethylbenzoat (X-Tend 226) oder Ölkomponenten der Marke Cosmacol wie Dimyristyl Tartrate, Tri C14-C15 Alkyl Citrate, C12-C13 Alkyl Lactate, Tridecyl Salicylate, C12-C13 Alkyl Octanoate, C12-C13 Alkyl Malate, C12-C13 Alkyl Citrate, C12-C13 Alkyl Tartrate), oder polarprotische Hilfsstoffe (z.B. Propylenglycol, Glycerin, Isopropanol, Ethanol) oder sog. Lösungsvermittler (z.B. Butylphthalimide, Isopropylphthalimide, Dimethylisosorbide). Ganz besonders bevorzugte kosmetische Öle sind C12-C13 Alkyl Lactate, kommerziell erhältlich als Cosmacol ELI und Phenethylbenzoat, kommerziell erhältlich als X-Tend 226.

Gegenstand der vorliegenden Erfindung ist auch ein Verfahren zur Herstellung einer Zubereitung, wie zuvor beschrieben, dadurch gekennzeichnet, dass mindestens eine Verbindung der Formel I mit einem für topische Zubereitungen geeigneten Träger, der weiteren Selbstbräunungssubstanz, und optional mit Hilfs- und oder Füllstoffen vermischt wird. Geeignete Trägerstoffe sowie Hilfs- oder Füllstoffe sind im nachfolgenden Teil ausführlich beschrieben.

Die genannten Bestandteile der Zubereitung können in der üblichen Weise eingearbeitet werden, mit Hilfe von Techniken, die dem Fachmann wohl bekannt sind.

Geeignet sind Zubereitungen für eine äußerliche Anwendung, beispielsweise als Creme oder Milch (O/W, W/O, O/W/O, W/O/W), als Lotion oder Emulsion, in Form ölig-alkoholischer, ölig-wässriger oder wässrigalkoholischer Gele bzw. Lösungen auf die Haut aufgesprüht werden kann. Sie können als feste Stifte vorliegen oder als Aerosol konfektioniert sein.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole, Pflaster, Umschläge, Verbände und Sprays.

Bevorzugte Hilfsstoffe stammen aus der Gruppe der Konservierungsstoffe, Stabilisatoren, Lösungsvermittler, Färbemittel, Geruchsverbesserer, Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel und andere in der Kosmetik gewöhnlich verwendete Ingredienzien.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3 Butylglykol, Öle, insbesondere Baumwollsaatöl, Erdnussöl, Maiskeimöl, Olivenöl, Rizinusöl und Sesamöl, XTend 226 (L'Oréal), Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methylcyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C Zahl, z.B. mit Isopropanol, Propylenglykol oder Glycerin, oder Ester von Fettalkoholen mit Alkansäuren niedriger C Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C Atomen und gesättigten und/oder ungesättigten, verzweigten und/oder unverzweigten Alkoholen einer Kettenlänge von 3 bis 30 C Atomen, aus der Gruppe der Ester aus aromatischen Carbonsäure und gesättigten und/oder ungesättigten, verzweigten und/oder unverzweigten Alkoholen einer Kettenlänge von 3 bis 30 C Atomen.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C Zahl, sowie deren Ether, vorzugsweise Ethanol, Isopropanol, Propylenglykol, Glycerin, Ethylenglykol, Ethylenglykolmonoethyl oder -monobutylether, Propylenglykolmonomethyl, -monoethyl oder -monobutylether, Diethylenglykolmonomethyl oder -monoethylether und analoge Produkte, ferner Alkohole niedriger C Zahl, z. B. Ethanol, Isopropanol, 1,2 Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Als Emulgatoren können beispielsweise bekannte W/O- und O/W-Emulgatoren verwendet werden. Es ist vorteilhaft, weitere übliche Co-Emulgatoren in den bevorzugten O/W-Emulsionen zu verwenden.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Weitere wichtige Merkmale und Vorteile der Erfindung ergeben sich aus den Unteransprüchen und aus den Beispielen.

Die Beispiele sollen die vorliegende Erfindung näher erläutern, ohne deren Umfang zu beschränken.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

### Beispiele:

### Beispiel 1:

### Synthese von Benzylharnstoff (Ia)

In einem 50mL-Rundkolben werden nacheinander 6,00g Benzylamin (56mmol) und 13,45g Harnstoff (224mmol) eingewogen. Anschließend werden 25mL Wasser über einen Messkolben hinzugefügt. Die Reaktionslösung wird 5 Minuten gerührt, mit 0,1-0,5mL konzentrierter Salzsäure versetzt und 3 Stunden am Rückfluss gekocht.

Das Reaktionsgemisch wird danach abgekühlt und bei Raumtemperatur kristallisiert das Produkt aus. Die Reaktionsmischung wird mit 50mL Wasser versetzt und der Niederschlag wird abfiltriert. Es wird mit 4x15mL Wasser, sowie 3x10mL EtOH gewaschen. Das Rohprodukt wird im Vakuumtrockenschrank (50mbar) bei 40°C getrocknet.

Ausbeute: 6,81g (82% der Theorie).
1H-NMR (500MHz, DMSO) δ[ppm] = 4,19 (d, 2H, CH₂-NH); 5,51 (s, 2H, NH₂); 6,41 (t, 1H, NH); 7,28 (ddt, 5H, Aromat).

### Beispiel 2:

### Synthese von (4-Methoxy-benzyl)-harnstoff (Ib)

Analog zu Beispiel 1 werden 1,50g 4-Methoxybenzylamin (10,72mmol) und 2,57g Harnstoff (42,86mmol) umgesetzt. Das Rohprodukt wird im Verhältnis 1:6 (Substanz: Lösemittel) aus Ethanol umkristallisiert.
Ausbeute: 0,72 g (37% der Theorie).
1H-NMR (500MHz, DMSO) δ[ppm] = 3,72 (s, 3H, CH₃-O); 4,10 (d, 2H, CH₂-NH); 5,46 (s, 2H, NH₂); 6,30 (t, 1H, NH) 6,86 (d, 2H, Aromat); 7,17 (d, 2H, Aromat).

### Beispiel 3:

### Synthese von Benzo[1,3]dioxol-5ylmethyl-harnstoff (Ic)

Analog zu Beispiel 1 werden 2,50g Piperonylamin (16,04mmol) und 3,85g Harnstoff (64,17mmol) umgesetzt.
Ausbeute: 2,87 g (88% der Theorie).
1H-NMR (500MHz, DMSO) δ[ppm] = 4,09 (d, 2H, CH₂-NH); 5,98 (s, 2H, O-CH₂-O); 5,48 (s, 2H, NH₂); 6,33 (t, 1H, NH) 6,72 (d, 1H, Aromat); 6,84 (dt, 2H, Aromat).z

### Beispiel 4:

### Synthese von (4-Hydroxy-3-methoxy-benzyl)-harnstoff (Id)

Analog zu Beispiel 1 werden 2,00g 4-(Aminomethyl)-2-methoxyphenol (10,44mmol) und 2,00g Harnstoff (33,30mmol) umgesetzt.
Ausbeute: 1,56 g (76% der Theorie).
1H-NMR (500MHz, DMSO) δ[ppm] = 3,75 (s, 3H, CH₃-O); 4,07 (s, 2H, CH₂-NH); 5,48 (s, 2H, NH₂); 8,75 (s, 1H, NH); 6,65 (d, 1H, Aromat) 6,72 (d, 1H, Aromat); 6,82 (s, 1H, Aromat); 12,5 (s, 1H, OH-Aromat)

### Beispiel 5:

### Synthese von (3,4-Dimethoxy-benzyl)-harnstoff (Ie)

Analog zu Beispiel 1 werden 2,50g 3,4-Dimethoxybenzylamin (14,50mmol) und 3,48g Harnstoff (58mmol) umgesetzt.
Ausbeute: 2,15 g (71% der Theorie).
1H-NMR (500MHz, DMSO) δ[ppm] = 3,73 (2s, 6H, CH₃-O); 4,10 (d, 2H, CH₂-NH); 5,47 (s, 2H, NH₂); 6,30 (t, 1 H, NH) 6,75-6,90 (2d, 1s, 3H, Aromat).

### Beispiel 6:

### Evaluierung der Melanin-Synthese in einem Zellkultur-Modell enthaltend zwei Zelltypen (Ko-Kultur), zum Einen normale humane epidermale Keratinozyten (NHEK) und zum Anderen normale humane epidermale Melanocyten, leicht pigmentiert (NHEM-LP)

Die beiden Zelltypen NHEK und NHEM-LP (NHEK:NHEM-LP) werden in einem Verhältnis von 2:1 eingesetzt.

Das Kulturmedium besteht aus den Medien Keratinocyte-SFM (2 Volumenanteile) und Medium M254 (1 Volumenanteil).

Keratinocyte-SFM enthält den epidermalen Wachstumsfaktor (EGF) mit 0,25 ng/ml, Pituitary-Extrakt (PE) mit 25 µg/ml und Gentamycin mit 25 µg/ml und wurde von der Firma Thermo Fisher Scientific bezogen.

M254 enthält PMA-freies HMGS-2, Insulin mit 5 µg/ml, Penicillin 50 U/ml, Streptomycin in 50µg/ml und Gentamycin mit 25 µg/ml und wurde von der Firma Thermo Fisher Scientific bezogen.

IBMX bezeichnet die Verbindung 3-Isobutyl-1-methylxanthin.

### Zellkultur und Behandlung

NHEK und NHEM-LP wurden in Mikrotiterplatten (24-well plates) für 24 Stunden im Kulturmedium inkubiert (37°C, 5% CO₂). Das Kulturmedium wurde entfernt und durch ein Assay-Medium ersetzt, welches das Kulturmedium und die zu testenden Verbindungen Ia, Ib und Ic oder auch keine Testsubstanz enthielt, die Referenz L-Tyrosin (1mM), IBMX mit 200µM oder die Lösemittelkontrolle THF mit 0,1%, THF mit 0,15% enthielt. Die Verbindungen Ia, Ib und Ic werden als Lösung (0.15% in THF) zugegeben. Die Zellen wurden nach Austausch des Assay-Mediums noch einmal für 240 Stunden (10 Tage) inkubiert. Am 3. und 7. Tag wurde noch einmal Assay-Medium zugegeben. Alle experimentellen Untersuchungen wurden mit einer 3-fachen Bestimmung durchgeführt. Nach Ende der Inkubation wurde durch Zelllyse mit 0,5 N NaOH-Lösung das Melanin extrahiert. Die optische Dichte (OD) jeder Probe wurde bei 405 nm gemessen. Die Melanin-Quantität wurde aus Melanin-Standards berechnet (Standardkurve 0,39 bis 100 µg/ml Melanin).

### Ergebnis:

Keine der Lösemittelkontrollen zeigte einen Einfluss auf die Melanin-Synthese.

Die Verbindung Ia, die mit 45 µM getestet wurde, stimuliert die Melanin-Synthese um 17%.

Die Verbindung Ib, die mit 45 µM getestet wurde, stimuliert die Melanin-Synthese um 18%.

Die Verbindung Ic, die mit 20 µM und mit 30 µM getestet wurde, zeigt einen konzentrationsabhängigen stimulierenden Effekt auf die Melanin-Synthese um 23% bzw. 44%.

### Beispiel 7:

### Evaluierung der Bräunungseigenschaften in vitro auf rekonstruierter Epidermis

Die Verbindung Ic wird in dieser Studie auf deren Bräunungseigenschaften untersucht, wobei diese *in vitro* auf 3D melanisierter rekonstruierter humanen Epidermis (RHEs-MEL = 3D melanized reconstructed human epidermis) untersucht wird. Als positive Kontrolle wird die Verbindung IBMX (3-Isobutyl-1-methylxanthin) 100µM eingesetzt. Die Verbindung Ic wird 25µM und 12,5 µM eingesetzt. Als negative Kontrolle gilt die unbehandelte rekonstruierte Haut. Die *in vitro* Haut wird 10 Tage systemisch mit den zu untersuchenden Verbindungen in einem Kulturmedium behandelt und danach analysiert.

### Ergebnis:

Die Verbindung Ic weisst vergleichbar gute Eigenschaften wie IBMX bei 100µM auf. Dies ist für beide Konzentrationen ersichtlich, sowohl in der Reduzierung des ITA-Wertes (ITA = Individual Typology Angle), des visuellen Farbunterschieds sowie der erhöhten Menge an Melanin.

### Beispiel 8: O/W-Formulierung

| **Bestandteile /Handelsname** | **Bezugsquelle** | **INCI** | **[Gew-%]** |
|---|---|---|---|
| **A** | | | |
| Marlipal 1618/11 | (1) | CETEARETH-11 | 3 |
| Lanette O | (2) | CETEARYLALCOHOL | 7 |
| Luvitol EHO | (3) | CETEARYLOCTANOATE | 5 |
| Tegosoft TN | (4) | C12-15 ALKYLBENZOATE | 2.5 |
| Miglyol 812 N | (1) | CAPRYLIC/CAPRIC TRIGLYCERIDE | 2.5 |
| Propyl-4-hydroxybenzoat | (5) | PROPYLPARABEN | 0.05 |
| **Verbindung Ia, Ib, Ic, Id oder Ie** | | | 0.5 |

| **B** | | | |
|---|---|---|---|
| 1,2-Propandiol | (5) | PROPYLENE GLYCOL | 4 |
| Methyl-4-hydroxybenzoat | (5) | METHYLPARABEN | 0.15 |
| Wasser, demineralisiert | | AQUA (WATER) | ad 100 |
| Wasser, demineralisiert | | | 10 |
| **Gesamt** | | | **100.00** |

### Herstellungsverfahren:

Zunächst wird die Phase A auf 75°C und die Phase B auf 80 °C erwärmt. Danach wird Phase B unter Rühren langsam zu Phase A gegeben und solange gerührt, bis eine homogene Mischung entsteht.

### Bezugsquellen:

(1) Sasol Germany GmbH (2) Cognis GmbH (3) BASF SE (4) Evonik Goldschmidt GmbH (5) Merck KGaA/Rona®

### Beispiel 9: O/W-Formulierung

| **Bestandteile / Handelsname** | **Bezugsquelle** | **INCI** | **[Gew-%]** |
|---|---|---|---|
| **A** | | | |
| Tego Care 150 | (1) | GLYCERYL STEARATE, STEARETH-25, CETETH-20, STEARYL ALCOHOL | 8 |
| Lanette O | (2) | CETEARYL ALCOHOL | 1.5 |
| Luvitol EHO | (3) | CETEARYL OCTANOATE | 5 |
| Miglyol 812 N | (4) | CAPRYLIC/CAPRIC TRIGLYCERIDE | 5 |
| Paraffin liquid | (5) | PARAFFINUM LIQUIDUM (MINERAL OIL) | 3 |
| AbilWax 2434 | (1) | STEAROXY DIMETHICONE | 1.6 |
| Dow Corning 200 Fluid (350 cs) | (6) | DIMETHICONE | 0.5 |
| Propyl-4-hydroxybenzoat | (5) | PROPYLLPARABEN | 0.05 |
| 5,7-Dihydroxy-2-methyl-chromon | (5) | | 0.2 |

| **B** | | | |
|---|---|---|---|
| 1,2-Propandiol | (5) | PROPYLENE GLYCOL | 3 |
| Methyl-4-hydroxybenzoat | (5) | METHYLPARABEN | 0.15 |
| Wasser, entmineralisiert | | AQUA (WATER) | ad 100 |

| **C** | | | |
|---|---|---|---|
| Probiol L 05018 (Leere Liposomen) | (7) | AQUA, ALCOHOL DENAT, LECITHIN, GLYCERINE, DISODIUM PHOSPHATE | 5 |
| Wasser, entmineralisiert | | AQUA (WATER) | 10.00 |
| Verbindung Ia, Ib, Ic, Id oder Ie | | | 0.2 |
| **Gesamt** | | | **100.00** |

### Herstellungsverfahren:

Zunächst werden die Phasen A und B auf 80 °C erwärmt. Danach wird Phase B unter Rühren langsam zu Phase A gegeben und homogenisiert. Dann wird abgekühlt und die Phase C bei 40°C zugegeben.

### Bezugsquellen:

(1) Evonik Goldschmidt GmbH, (2) Cognis GmbH, (3) BASF SE, (4) Sasol Germany GmbH, (5) Merck KGaA/Rona®, (6) Dow Corning, (7) Kuhs GmbH & Co. KG

### Beispiel 10: W/O-Formulierung

| **Bestandteile / Handelsname** | **Bezugsquelle** | **INCI** | **[Gew-%]** |
|---|---|---|---|
| **A** | | | |
| Dow Corning 3225 C | (1) | CYCLOMETHICONE, DIMETHICONE COPOLYOL | 23.6 |
| Propyl-4-hydroxybenzoat | (2) | PROPYLPARABEN | 0.05 |
| Verbindung Ia, Ib, Ic, Id oder Ie | | | 0.1 |

| **B** | | | |
|---|---|---|---|
| | | | |
| Methyl-4-hydroxybenzoat | (2) | METHYLPARABEN | 0.15 |
| 1,2-Propandiol | (2) | PROPYLENE GLYCOL | 35.9 |
| Wasser, entmineralisiert | | AQUA (WATER) | ad 100 |
| | | | |
| **Gesamt** | | | **100.00** |

### Herstellungsverfahren:

Zunächst wird die Phase B aufgelöst und dann wird sie zu Phase A gegeben. Der pH-Wert wird mit Natronlauge bzw. Citronensäure auf den Wert pH = 6.0 eingestellt.

### Bezugsquellen:

(1) Dow Corning (2) Merck KGaA/Rona®

### Beispiel 11: O/W Anti-aging Creme mit UV A/B Schutz

| **Bestandteile / Handelsname** | **Bezugsquelle** | **INCI** | **[Gew-%]** |
|---|---|---|---|
| **A** | | | |
| Eusolex® 2292 | (1) | ETHYLHEXYL METHOXYCINNAMATE, BHT | 3 |
| Eusolex® 4360 | (1) | BENZOPHENONE-3 | 0.5 |
| Tego Care 150 | (2) | GLYCERYL STEARATE, STEARETH-25, CETETH-20, STEARYL ALCOHOL | 8 |
| Lanette O | (3) | CETEARYL ALCOHOL | 1.5 |
| Luvitol EHO | (4) | CETEARYL OCTANOATE | 5 |
| Miglyol 812 N | (5) | CAPRYLIC/CAPRIC TRIGLYCERIDE | 5 |
| Paraffin liquid | (1) | PARAFFINUM LIQUIDUM (MINERAL OIL) | 3 |
| Abil-Wax 2434 | (2) | STEAROXY DIMETHICONE | 1.6 |
| Dow Corning 200 Fluid (350 cs) | (6) | DIMETHICONE | 0.5 |
| Propyl-4-hydroxybenzoat | (1) | PROPYLPARABEN | 0.05 |
| Verbindung Ia, Ib, Ic, Id oder Ie | | | 1 |

| **B** | | | |
|---|---|---|---|
| 1,2-Propandiol | (1) | PROPYLENE GLYCOL | 3 |
| Methyl-4-hydroxybenzoat Natriumsalz | (1) | SODIUM METHYLPARABEN | 0.17 |
| Wasser, demineralisiert | | AQUA (WATER) | ad 100 |
| | | | |
| **Gesamt** | | | **100.00** |

### Herstellungsverfahren:

Zunächst werden die Phasen A und B getrennt gemischt und auf 80°C erwärmt. Danach wird Phase B langsam unter Rühren zu Phase A gegeben. Es wird homogenisiert auf Raumtemperatur abgekühlt.

Bezugsquellen: (1) Merck KGaA/Rona®, (2) Evonik Goldschmidt GmbH, (3) Cognis GmbH, (4) BASF AG, (5) Sasol Germany GmbH, (6)

## Patentansprüche

1. Verwendung von Verbindungen der Formel I, 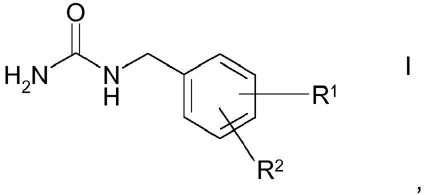 wobei
R¹ und R² unabhängig voneinander für
-H,
-OH,
-geradkettiges oder verzweigtes O-(C₁- bis C₆-Alkyl) stehen,
wobei R¹ und R² auch zusammen einen unsubstituierten oder substituierten fünfgliedrigen Ring bilden können, wobei ein oder zwei nicht benachbarte CH₂-Gruppen durch O ersetzt sein können und der durch mindestens eine geradkettige oder verzweigte Alkylgruppe mit 1 bis 6 C-Atomen substituiert sein kann,
und/oder deren Salze, Tautomere, Konformere und/oder Solvate, einschließlich deren Mischungen in allen Verhältnissen, als Selbstbräunungssubstanz.

2. Verwendung nach Anspruch 1, **dadurch gekennzeichnet, dass** R¹ in Verbindungen der Formel I für H, OH oder eine geradkettige oder verzweigte Alkoxygruppe mit 1 bis 4 C-Atomen steht.

3. Verwendung nach Anspruch 1 oder 2, **dadurch gekennzeichnet, dass** R² in Verbindungen der Formel I für H, OH oder eine geradkettige oder verzweigte Alkoxygruppe mit 1 bis 4 C-Atomen steht.

4. Verwendung nach Anspruch 1, **dadurch gekennzeichnet, dass** R¹ und R² in Verbindungen der Formel I zusammen einen fünfgliedrigen Ring enthaltend zwei O-Atome bilden, der durch eine oder zwei geradkettige oder verzweigte Alkylgruppe(n) mit 1 bis 6 C-Atomen substituiert sein kann.

5. Verwendung nach einem oder mehreren der Ansprüche 1 bis 4, **dadurch gekennzeichnet, dass** die Verbindung der Formel I ausgewählt ist aus den Verbindungen der Formel Ia bis Ie

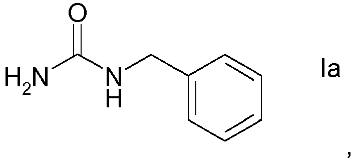

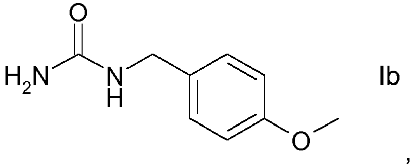

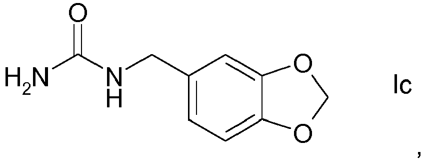

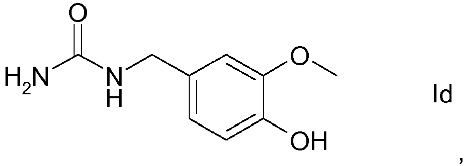

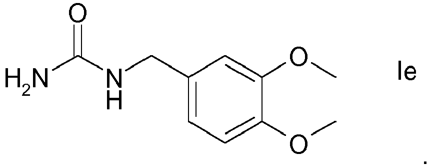

6. Zubereitung enthaltend einen für topische Anwendungen geeigneten Träger, mindestens eine Verbindung der Formel I in einer Menge von 0,01 bis 10 Gew.-%, 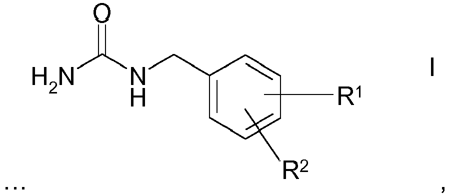 wobei
R¹ und R² unabhängig voneinander für
-H,
-OH,
-geradkettiges oder verzweigtes O-(C₁- bis C₆-Alkyl) stehen,
wobei R¹ und R² auch zusammen einen unsubstituierten oder substituierten fünfgliedrigen Ring bilden können, wobei ein oder zwei nicht benachbarte CH₂-Gruppen durch O ersetzt sein können und der durch mindestens eine geradkettige oder verzweigte Alkylgruppe mit 1 bis 6 C-Atomen substituiert sein kann
und/oder deren Salze, Tautomere, Konformere und/oder Solvate, einschließlich deren Mischungen in allen Verhältnissen, und mindestens eine weitere Selbstbräunungssubstanz.

7. Verfahren zur Herstellung einer Zubereitung nach Anspruch 6, **dadurch gekennzeichnet, dass** mindestens eine Verbindung der Formel I mit einem für topische Anwendungen geeigneten Träger, der weiteren Selbstbräunungssubstanz, und optional mit anderen Aktiv- oder Hilfsstoffen, vermischt wird.

8. Verbindungen der Formel I, 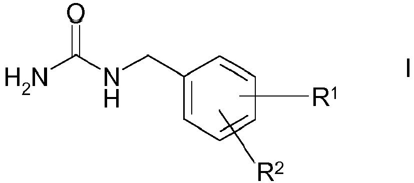 wobei
R¹ und R² unabhängig voneinander für
-H,
-OH,
-geradkettiges oder verzweigtes O-(C₁- bis C₆-Alkyl) stehen,
wobei R¹ und R² auch zusammen einen unsubstituierten oder substituierten fünfgliedrigen Ring bilden können, wobei ein oder zwei nicht benachbarte CH₂-Gruppen durch O ersetzt sein können und der durch mindestens eine geradkettige oder verzweigte Alkylgruppe mit 1 bis 6 C-Atomen substituiert sein kann,
und/oder deren Salze, Tautomere, Konformere und/oder Solvate, einschließlich deren Mischungen in allen Verhältnissen, zur Verwendung zur Steigerung der Melaninsynthese, zur Verbesserung des Melanintransports und/oder zur Verbesserung der Verteilung von Melanin in suprabasalen Schichten.

9. Verbindungen nach Anspruch 8, ausgewählt aus den Verbindungen der Formel Ia bis Ie

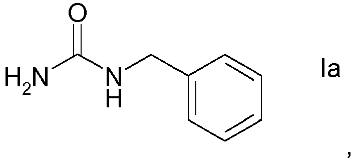

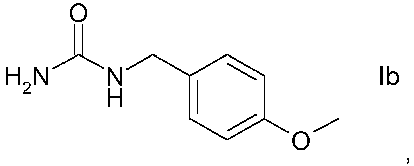

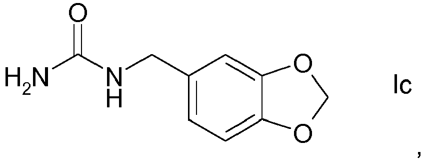

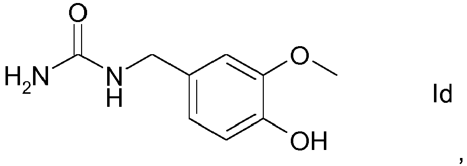

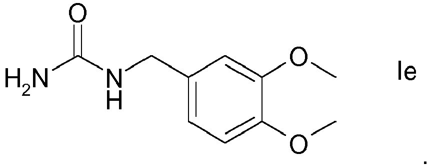

## Claims

1. Use of compounds of the formula I, 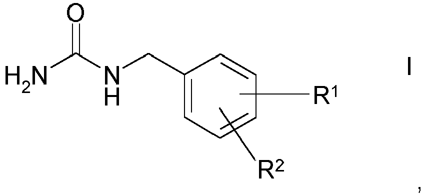 where
R¹ and R² stand, independently of one another, for
-H,
-OH,
-straight-chain or branched O-(C₁- to C₆-alkyl),
where R¹ and R² may also together form an unsubstituted or substituted five-membered ring, in which one or two non-adjacent CH₂ groups may be replaced by O and which may be substituted by at least one straight-chain or branched alkyl group having 1 to 6 C atoms,
and/or salts, tautomers, conformers and/or solvates thereof, including mixtures thereof in all ratios, as self-tanning substance.

2. Use according to Claim 1, **characterised in that** R¹ in compounds of the formula I stands for H, OH or a straight-chain or branched alkoxy group having 1 to 4 C atoms.

3. Use according to Claim 1 or 2, **characterised in that** R² in compounds of the formula I stands for H, OH or a straight-chain or branched alkoxy group having 1 to 4 C atoms.

4. Use according to Claim 1, **characterised in that** R¹ and R² in compounds of the formula I together form a five-membered ring containing two O atoms, which may be substituted by one or two straight-chain or branched alkyl group(s) having 1 to 6 C atoms.

5. Use according to one or more of Claims 1 to 4, **characterised in that** the compound of the formula I is selected from the compounds of the formulae Ia to le

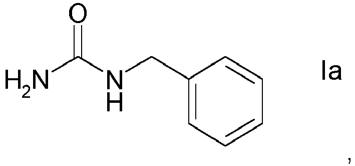

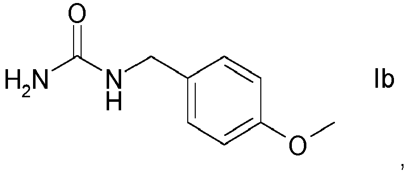

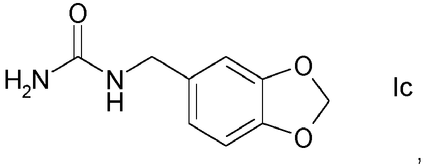

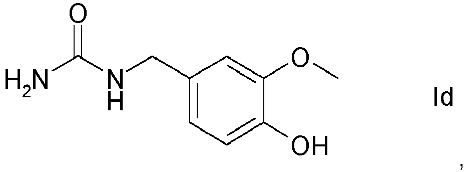

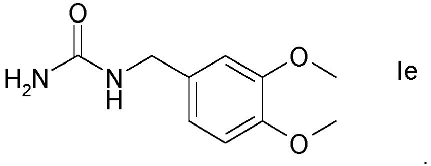

6. Preparation comprising a vehicle which is suitable for topical applications, at least one compound of the formula I in an amount of 0.01 to 10% by weight, 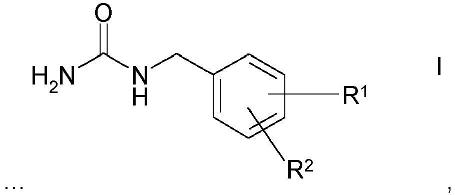 where
R¹ and R² stand, independently of one another, for
-H,
-OH,
-straight-chain or branched O-(C₁- to C₆-alkyl),
where R¹ and R² may also together form an unsubstituted or substituted five-membered ring, in which one or two non-adjacent CH₂ groups may be replaced by O and which may be substituted by at least one straight-chain or branched alkyl group having 1 to 6 C atoms,
and/or salts, tautomers, conformers and/or solvates thereof, including mixtures thereof in all ratios, and at least one further self-tanning substance.

7. Process for preparing a preparation according to Claim 6, **characterised in that** at least one compound of the formula I is mixed with a vehicle which is suitable for topical applications, the further self-tanning substance and optionally with other active substances or assistants.

8. Compounds of the formula I, 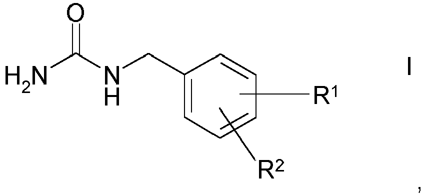 where
R¹ and R² stand, independently of one another, for
-H,
-OH,
-straight-chain or branched O-(C₁- to C₆-alkyl),
where R¹ and R² may also together form an unsubstituted or substituted five-membered ring, in which one or two non-adjacent CH₂ groups may be replaced by O and which may be substituted by at least one straight-chain or branched alkyl group having 1 to 6 C atoms,
and/or salts, tautomers, conformers and/or solvates thereof, including mixtures thereof in all ratios, for use for increasing melanin synthesis, for improving melanin transport and/or for improving the distribution of melanin in suprabasal layers.

9. Compounds according to Claim 8, selected from the compounds of the formulae Ia to le

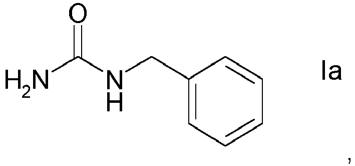

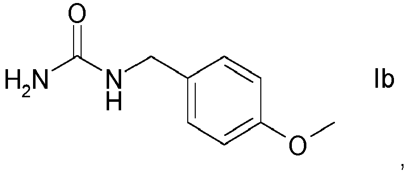

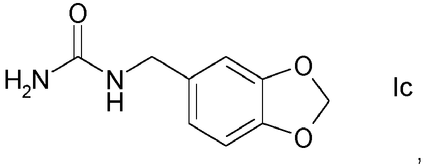

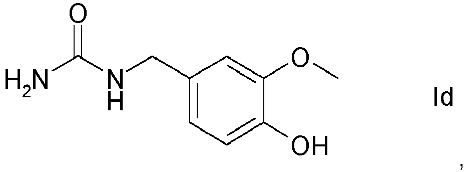

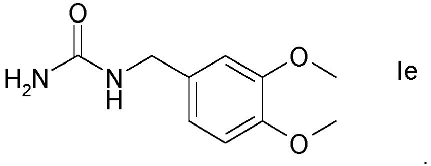

## Revendications

1. Utilisation de composés de formule I, 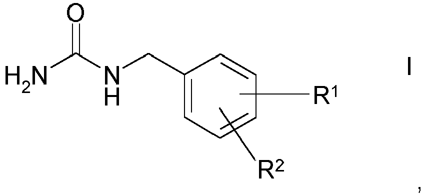 dans laquelle
R¹ et R² représentent, indépendamment l'un de l'autre,
- H,
- OH,
- O-(C₁- à C₆-alkyl) à chaîne linéaire ou ramifiée,
où R¹ et R² peuvent également former ensemble un cycle de cinq chaînons non substitué ou substitué, dans lequel un ou deux groupements CH₂ non adjacents peuvent être remplacés par O et qui peut être substitué par au moins un groupement alkyle à chaîne linéaire ou ramifiée ayant de 1 à 6 atomes de C,
et/ou les sels, tautomères, conformères et/ou solvates de ceux-ci, y compris des mélanges de ceux-ci en toutes proportions, comme substance auto-bronzante.

2. Utilisation selon la revendication 1, **caractérisée en ce que** R¹ dans les composés de formule I représente H, OH ou un groupement alcoxy à chaîne linéaire ou ramifiée ayant de 1 à 4 atomes de C.

3. Utilisation selon la revendication 1 ou 2, **caractérisée en ce que** R² dans les composés de formule I représente H, OH ou un groupement alcoxy à chaîne linéaire ou ramifiée ayant de 1 à 4 atomes de C.

4. Utilisation selon la revendication 1, **caractérisée en ce que** R¹ et R² dans les composés de formule I forment ensemble un cycle de cinq chaînons contenant deux atomes de O, qui peut être substitué par un ou deux groupement(s) alkyle à chaîne linéaire ou ramifiée ayant de 1 à 6 atomes de C.

5. Utilisation selon l'une ou plusieurs parmi les revendications 1 à 4, **caractérisée en ce que** le composé de formule I est choisi parmi les composés de formules la à le

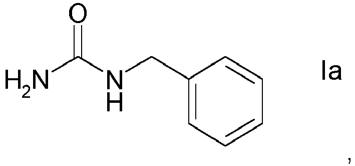

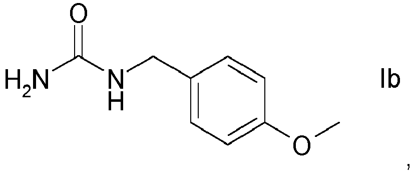

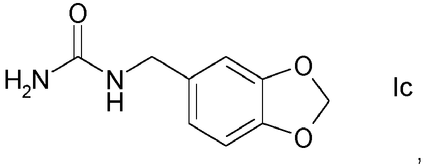

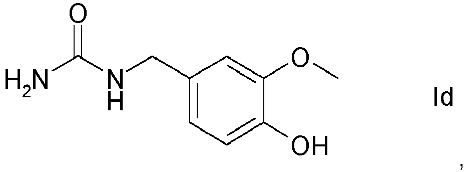

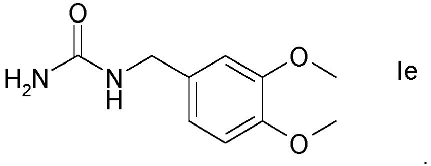

6. Préparation comprenant un véhicule qui est convenable pour des applications topiques, au moins un composé de formule I selon une quantité allant de 0,01 à 10% en poids, 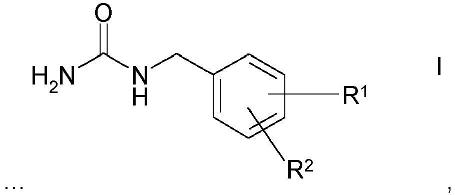 dans laquelle
R¹ et R² représentent, indépendamment l'un de l'autre,
- H,
- OH,
- O-(C₁- à C₆-alkyl) à chaîne linéaire ou ramifiée,
où R¹ et R² peuvent également former ensemble un cycle de cinq chaînons non substitué ou substitué, dans lequel un ou deux groupements CH₂ non adjacents peuvent être remplacés par O et qui peut être substitué par au moins un groupement alkyle à chaîne linéaire ou ramifiée ayant de 1 à 6 atomes de C,
et/ou les sels, tautomères, conformères et/ou solvates de celui-ci, y compris des mélanges de ceux-ci en toutes proportions, et au moins une autre substance auto-bronzante.

7. Procédé de préparation d'une préparation selon la revendication 6, **caractérisé en ce qu'**au moins un composé de formule I est mélangé avec un véhicule qui est convenable pour des applications topiques, l'autre substance auto-bronzante et éventuellement avec d'autres substances actives ou adjuvants.

8. Composés de formule I, 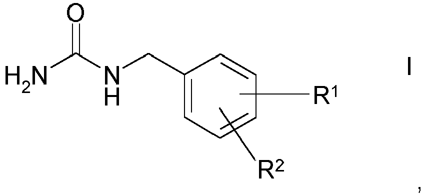 dans laquelle
R¹ et R² représentent, indépendamment l'un de l'autre,
- H,
- OH,
- O-(C₁- à C₆-alkyl) à chaîne linéaire ou ramifiée,
où R¹ et R² peuvent également former ensemble un cycle de cinq chaînons non substitué ou substitué, dans lequel un ou deux groupements CH₂ non adjacents peuvent être remplacés par O et qui peut être substitué par au moins un groupement alkyle à chaîne linéaire ou ramifiée ayant de 1 à 6 atomes de C,
et/ou les sels, tautomères, conformères et/ou solvates de ceux-ci, y compris des mélanges de ceux-ci en toutes proportions, pour une utilisation destinée à augmenter la synthèse de mélanine, à améliorer le transport de la mélanine et/ou à améliorer la distribution de la mélanine dans les couches suprabasales.

9. Composés selon la revendication 8, choisis parmi les composés de formules la à le
